# EUROPÄISCHE PATENTSCHRIFT

(11) **EP 0 826 359 B1**
(45) Veröffentlichungstag und Bekanntmachung des Hinweises auf die Patenterteilung: **01.03.2006**
(21) Anmeldenummer: 97115268.1
(22) Anmeldetag: 03.09.1997
(51) Int. Cl.: A61K 6/083, A61K 6/09, C09J 133/00, C09J 175/04

(54) **Adhäsiv als Haftvermittler zwischen elastischen und starren Materialien**
Adhesive as a coupling agent between elastic and rigid materials
Adhésif comme agent de couplage entre des matériaux élastiques et rigides

(30) Priorität: 03.09.1996 DE 19635696; 25.03.1997 DE 19712341
(43) Veröffentlichungstag der Anmeldung: 04.03.1998
(73) Patentinhaber: VOCO GmbH, 27472 Cuxhaven (DE)
(72) Erfinder: Harenbrock, Michael Dr., 27472 Cuxhaven (DE); Plaumann, Manfred Thomas, 27476 Cuxhaven (DE)
(74) Vertreter: Hauck, Graalfs, Wehnert, Döring, Siemons

(56) Entgegenhaltungen:
- EP-A- 0 632 060
- DE-A- 4 414 837
- DATABASE WPI Section Ch, Week 199215 Derwent Publications Ltd., London, GB; Class A14, AN 1992-121117 XP002131222 & JP 04 068007 A (FUJI SYSTEMS KK), 3. März 1992 (1992-03-03)

## Beschreibung

Die Erfindung bezieht sich auf ein Adhäsiv als Haftvermittler zwischen elastischen und starren Materialien.

Ein wichtiges werkstoffkundliches Problem besteht in der Verbindung zwischen chemisch unterschiedlichen Massen, wie z.B. elastischem Material und starren Oberflächen wie Glas, Metall oder Kunststoff. In der Zahnmedizin stellt sich dieses Problem beispielsweise wenn Metall oder Kunststoff mit Silikon verklebt werden soll. Diese Problematik tritt vornehmlich bei der Haftung von Abformmaterialien auf metallenen bzw. methacrylathaltigen (individuellen, d.h. speziell für den Patienten angefertigten) Abformlöffeln oder bei der Haftung von Unterfütterungsmaterialien auf Prothesenkunststoffen zur permanenten Unterfütterung herausnehmbaren Zahnersatzes auf.

Die Abformung dient in der zahnärztlichen Prothetik zur Herstellung eines Negativs der Zahnhartsubstanz sowie des umgebenden Gewebes. Das aus diesem Negativ durch Ausgießen mit Gips oder geeigneten Kunststoffen angefertigte sogenannte Meistermodell bildet die Grundlage für die prothetische Versorgung des Patienten. Da die Paßgenauigkeit der zahntechnischen Arbeit maßgeblich von der Präzision des Modells und somit der Abformung abhängt, muß die Abdrucknahme exakt und zuverlässig erfolgen. Um dies zu gewährleisten, muß der Abformwerkstoff vor dem Entfernen der Abformung aus dem Mund und während der Modellherstellung eine feste Verbindung zum Abformmasseträger aufweisen. Mechanische Beanspruchungen, die durch das Abziehen über unter sich gehende Stellen oder durch Haftung an Zähnen sowie Weichteilen bedingt sind, wirken sich nicht nur auf das Material selbst, sondern auch auf die Haftung des Materials am Abformlöffel aus. Behält die Abformmasse nicht ihren Platz im Löffel oder kehrt sie nicht vollkommen in ihre Ausgangslage zurück, so wird die gesamte Abformung verzerrt. Die zahntechnische Arbeit wird dadurch ungenau und muß gegebenenfalls wiederholt werden.

Um die Haftung zu erhöhen, kommen perforierte oder mit Retentionskanten versehene Abformlöffel zum Einsatz. Hier zeigen neueste Untersuchungen, daß die Fehlerrate bei Abdrucknahmen mit perforierten Abformlöffeln höher ist als beim Einsatz unperforierter Abformlöffel, da ein vollständiges Rückstellen der elastischen Materialien für eine exakte Abformung unabdingbar ist. Dieses Rückstellen kann aber durch das Durchziehen der Abfomunasse durch die Perforationslöcher negativ beeinträchtigt werden. Zudem wirken Löffelperforationen klinisch unkalkulierbar und können zu verfälschten Modellen führen. Untersuchungen von A. H. L. Tjan et al. [J. Prosth. Dent. 58, 175 (1987)] zeigen eindeutig, daß Perforationen den unveränderten Sitz der Abformmassen über einen längeren Zeitraum nicht gewährleisten. Aus diesem Grund ist die Verwendung perforierter Abformlöffel umstritten.

Die Zuverlässigkeit der Haftung und die Haftkräfte von Abformmaterial auf mit Retentionskanten versehenen Abformlöffeln wird gegenüber den perforierten Abformlöffeln zwar als höher beurteilt, dennoch ist auch deren Verwendung unzulänglich.

Ziel für die Metall/Silikon- oder Kunststoff/Silikon-Verbundverfahren in der Zahnmedizin ist es, eine dem Metall/Keramik-Verbund vergleichbare chemische Anbindung dieser Werkstoffe zu erreichen, da nur so eine kraftschlüssige Verbindung der Werkstoffe entsteht. Dies wird zum Teil durch eine geeignete Oberflächenkonditionierung in unterschiedlichen Verfahren realisiert. Diese Verfahren lassen sich je nach Art der aufgebrachten Verbundschicht in anorganische Verfahren, wie die Silikatisierungsmethoden Silicoater®, Silicoater®-MD, Rocatec® und in organische Verbundverfahren, wie Spektra Link, Zeta HLC-Bond oder Kevloc®, einteilen [Dental-Labor XLIV 12, 2045 (1996)]. Bei allen diesen Verfahren, beispielsweise in den Druckschriften US 4 364 731, DE 3 403 894 oder DE 3 802 043 beschrieben, ist es Stand der Technik, daß eine mechanische Oberflächenvorbehandlung durchgeführt wird oder eine zusätzliche Verbundschicht aufgebracht wird. Dies ist mit einem erheblichen apparativen als auch zeitlichen Aufwand verbunden. Eine Vorbehandlung eines metallenen Abformlöffels nach den oben angeführten Verfahren kann zudem zu einem extrem starken Verbund zwischen Abformmasse und Abformmassenträger führen. Dieser kann so stark sein, daß die Abdruckmasse nicht mehr vom Metall entfernt werden kann, so daß der Abformlöffel nicht mehr verwendet werden kann.

Nach dem Stand der Technik wird zur sicheren Haftvermittlung die Verwendung von Adhäsiven empfohlen, die einen zuverlässigen Verbund zwischen Abformmasse und Metall gewährleisten, die aber nach Beendigung der zahntechnischen Arbeit leicht mit einem handelsüblichen Lösemittel zu entfernen sind. Die zum heutigen Zeitpunkt auf dem Markt befindlichen Haftlacke verbessern die Haftung zwar nicht grundsätzlich, sind den Perforationen aber überlegen. Nach neuesten Untersuchungen [Swiss Dent 11-S, 55 (1996)] ist für das Gelingen einer verzerrungsfreien Abformung mit hoher Genauigkeit die Verwendung eines Adhäsivs sogar obligatorisch. Die Haftkräfte am Abformlöffel sind allerdings produktabhängig recht unterschiedlich. Bei allen Abformungen wird darauf hingewiesen, stets die Herstellerkombination Abformwerkstoff - Adhäsiv zu verwenden, um eine gute Haftung zu erreichen.

Eine willkürliche oder von den veränderlichen Anforderungen abhängige Materialkombination erzielt nicht den gewünschten Erfolg.

Ein weiterer Nachteil handelsüblicher Haftlacke ist deren sogenannter Kaugummi-Effekt [TZB 11, 426 (1996)]. Dieser Effekt täuscht mehr den unverrückbaren Sitz der Abformmasse durch das Adhäsiv vor, als ihn tatsächlich zu gewährleisten.

Aus klinischer Sicht sind für eine zuverlässige Haftvermittlung zwischen Metallabformlöffel und Abformmasse nach neuesten Untersuchungen von Welker, D. et al. [TZB 11, 426 (1996)] Haftkräfte, gemessen als Scherfestigkeit, von mindestens 40 N/cm² erforderlich. Eine Obergrenze der Haftvermittlung wird lediglich durch die Entfembarkeit des Abformmaterials vom Konfektionslöffel nach Beendigung der Arbeit definiert.

In der DE-PS 42 28 530 wird die Haftung zwischen Metall-, Keramik-, Glas-, Polymer-Kunststoff-Verbunden beschrieben, die aus der Verwendung eines 1-Komponentenhaftvermittlers, der keine zusätzlichen Haftvermittlerlösungen benötigt, resultieren. Nachteilig wirkt sich hierbei jedoch aus, daß zur Erzielung der gewünschten Haftung die Verbundschicht einer Wärmebehandlung unterzogen werden muß.

Neben der beispielhaft dargestellten zu verbessernden temporären Haftvermittlung zwischen Abformmaterialien und Metallen oder individuellen Abformlöffeln ist auch die permanente Haftung von Silikonmaterialien auf Prothesenkunststoffen ein weiteres wichtiges zahnmedizinisches Problem.

In den vergangenen Jahren sind zahlreiche Untersuchungen und Vorschläge gemacht worden, Kunststoffe haftfest mit Silikonmaterialien zu verbinden. Vor allem im Dentalbereich ist eine solche Haftvermittlung maßgeblich für das Gelingen der permanenten Unterfütterung von Prothesen, die vornehmlich aus Polymethyl(meth)acrylat (PMMA) bestehen, verantwortlich. Eine permanente Unterfütterung kann erforderlich werden, wenn eine Prothese einer veränderten Kiefersituation, z.B. durch Rückbildung des Kieferkamms, anzupassen ist. In bezug auf die Anforderungen eines dauerhaft weichbleibenden Uriterfiitterungsmaterials sind Silikone, aufgrund ihrer vorteilhaften Eigenschaften, wie dauerhafter Elastizität und Feuchtigkeitsbeständigkeit, besonders gut geeignet [Quintessenz Zahntech. 23, 5, 675-680, (1997)]. Da sie als primär weiche Werkstoffe nicht über zugesetzte Weichmacher oder monomere (Meth)acrylate verfügen, lassen sie sich zusätzlich als Prothesenschutzschicht für die Versorgung von Patienten einsetzen, bei denen eine Unverträglichkeit des Prothesenkunststoffes bekannt ist. Darüber hinaus lassen sich Silikone auch zur Unterstützung des Heilprozesses in der Implantologie verwenden. Als äußerst unbefriedigend erweist sich zur Zeit jedoch deren Haftung am Prothesenkunststoff.

Nach dem Stand der Technik werden für diese Zwecke meist Adhäsive verwendet, deren Nachteil darin besteht, daß das Unterfiitterungsmaterial frühestens nach einer Ablüftungszeit von ca. einer Stunde aufgetragen werden kann. Alternativ kann das Aufbringen des Haftvermittlers durch sogenanntes Einbrennen bei erhöhter Temperatur durchgeführt werden, wie es in der Patentanmeldung EP 0 349 897 beschrieben wird. Beide Vorgehensweisen sind mit hohem Zeitaufwand und Unannehmlichkeiten für den Patienten verbunden. Vorteilhaft wäre daher die Verwendung eines Haftvermittlers, der das sofortige Aufbringen des Unterfütterungsmaterials auf die aufgetragene Haftvermittlerschicht ermöglicht.

Neuere Entwicklungen, wie sie im Patent DE 44 14 837 beschrieben sind, tragen diesen Forderungen nur zum Teil Rechnung. Bei dem Haftsystem, das im Patent DE 44 14 837 beansprucht wird, handelt es sich um einen 2-komponentigen, mit Silan versetzten Reaktionsklebstoff, der nach Auftragen auf die vorbehandelte Prothesenoberfläche direkt mit einem additionsvemetzenden Silikon (A-Silikon) umgesetzt werden kann. Bei diesem Verfahren lassen sich jedoch Dosier-, Misch-, Verarbeitungs- und Anwendungsfehler nicht ausschließen. Darüber hinaus muß durch eine unvollständige Umsetzung der Reaktionsteilnehmer mit dem Vorhandensein von toxikologisch bedenklichen Restmonomeren, wie beispielsweise Isocyanaten, gerechnet werden. Für den Anwender wirkt sich zudem bei Verwendung eines 2-komponentigen Haftvermittlers der Zeitaufwand negativ aus.

In den Druckschriften DE 38 04 154, DE 44 14 982, EP 685 547 und EP 384 401 werden Silikon-, PMMA- oder Polyvinylacetat-Haftklebezusammensetzungen mit hoher Klebrigkeit beschrieben, die sich jedoch durch Aushärtungsmechanismen, Wärmebehandlungen und lange Einwirkzeiten der Klebstoffzusammensetzung nachteilig auf eine schnelle und somit patienten- sowie anwenderfreundliche Haftvermittlung auswirken. Bei den in diesen Druckschriften beschriebenen Haftvermittlern werden die reaktiven, zu einer Bindung zwischen Silikon und Kunststoff führenden chemischen funktionellen Gruppen erst während des Einwirkvorganges, z.B. durch Hydrolyse, gebildet.

In der Druckschrift US 3 785 054 wird ein Prothesenunterfütterungsmaterial beansprucht, in das die haftvermittelnden Substanzen unter hoher Temperatur (mindestens 60 °C) bereits mit eingearbeitet sind.

In der Druckschrift EP 632 060 wird ein auf einem (Meth)acrylat basierendes Adhäsiv beschrieben, das vornehmlich durch eine platinkatalysierte Hydrosilylierung hergestellt werden kann. Die in der Druckschrift beschriebenen Verbindungen und Herstellungsverfahren führen zu den bekanntermaßen unpolaren Silizium-Kohlenstoff Bindungen im Adhäsiv. Diese Bindungen sollten die Haftvermittlung gegenüber Wasserangriffen und Hydrolysespaltung stabiler machen [Silicones - Chemistry and Technology. Vulkan-Verlag Essen (1991)].

Handelsübliche Adhäsive für Unterfiitterungssilikone sind aufgrund ihres chemischen Aufbaus ausschließlich für eine Anwendung auf Prothesen aus PMMA geeignet. Da allergische Reaktionen gegen diese Kunststoffe bzw. einzelne Bestandteile daraus zunehmen, wird intensiv an der Herstellung von Prothesenkunststoffen gearbeitet, die aufgrund ihres chemischen Aufbaus nicht zu allergischen Reaktionen führen. Es handelt sich hierbei um Kunststoffe, z.B. aus einem Methacrytat/Vinylacetat/Vinylchlorid-Copolymer, aus Polyacetalen, Polyamiden, Polycarbonaten, Polyestern sowie compositähnlichen Urethandiacrylaten [Quintessenz Zahntechn. 23, 5, 707-720, (1997)]. Die Zugänglichkeit eines Haftvermittlers für die Unterfiitterung all dieser Kunststoffe mit Silikonen ist wünschenswert.

Aufgabe der Erfindung ist es deshalb, ein Adhäsiv zur Verfügung zu stellen, daß die Haftung zwischen elastischen und starren Materialien verbessert, das insbesondere
- zuverlässige Verbunde zwischen elastischen Materialien, z.B. additionsvemetzenden Silikonen, und jeweils unterschiedlichen starren Materialien ermöglicht,
- universell, bezogen auf die Haftkraft, die Haftzeit und die verwendeten Materialien, dem jeweiligen Haftproblem Rechnung tragen kann,
- hohe Haftkräfte ermöglicht, die nur von der Zerreißfestigkeit der Materialien begrenzt werden,
- einen Haftverbund ermöglicht, der bei Bedarf durch Anwendung eines Lösungsmittels einfach aufzuheben ist,
- das einfach und schnell zu applizieren ist.

Diese Aufgabe wird erfindungsgemäß durch das Verfahren gemäß Anspruch 1 gelöst. Vorteilhafte Ausgestaltungen des Verfahrens sind in den Ansprüche 2 bis 14 angegeben.

Ein Vorteil des nach dem erfindungsgemäßen Verfahren hergestellten Adhäsivs liegt in der sich schnell und zuverlässig ausbildenden chemischen Bindung zwischen den zu verbindenden Materialien. In dem erfindungsgemäßen Adhäsiv sind die zur Haftvermittlung benötigten reaktiven, zu einer haftfesten und kraftschlüssigen Bindung führenden chemischen Gruppen im Adhäsiv bereits enthalten, so daß die Einwirkzeit lediglich durch die Verdunstungsgeschwindigkeit des Lösemittels begrenzt wird. Die chemische Bindung führt zu hohen Haftwerten, die je nach Einsatzgebiet einzig durch die Zerreißfestigkeit der elastischen Materialien begrenzt werden. Bei dem Adhäsiv ergeben sich ohne einen aufwendigen Katalysator (vgl. EP 632 060) Silizium-Sauerstoff-Bindungen, die überraschenderweise ausreichend beständig für eine kraftschlüssige Haftvermittlung sind. Ein weiterer Vorteil besteht darin, daß die starre Oberfläche des zu verklebenden Materials nicht notwendigerweise vorbehandelt werden muß. Der Haftvermittler wird einfach auf die starre, unbehandelte Oberfläche aufgetragen. Nach dem Ablüften kann unmittelbar das Silikon aufgetragen werden. Eine zeit- und apparativaufwendige Wärmebehandlung entfällt ebenfalls. Es kommt somit bei Verwendung des erfindungsgemäßen Haftmittels zu einer anwendungsfreundlichen Zeitersparnis. Der Haftvermittler ist als 1-Komponentenmaterial anzuwenden. Gegenüber Mehrflaschenpräparaten beinhaltet das Einflaschenpräparat den Vorteil einer anwendungsfreundlichen Applikation und der Vermeidung von Mischungsfehlern.

Durch Variation der Bestandteile des nach dem erfindungsgemäßen Verfahren gemäß den Ansprüchen 1 bis 5 hergestellten Adhäsivs ist, wie in einem Baukastensystem (Adhäsivsystem), eine Anpassung des Adhäsivs an jede individuelle Anforderung bezüglich der Haftvermittlung möglich. D.h. zum einen, man erreicht eine zuverlässige und kraftschlüssige Haftung zwischen Silikonen und den gewünschten Materialien wie Metall, Kunststoff, Glas etc., zum anderen ist eine Variation der Dauerhaftigkeit der Haftung von permanent bis temporär einstellbar. Durch gezieltes Mischen der beanspruchten Adhäsivkomponenten wird eine individuell einstellbare Haftung zwischen elastischen und starren Materialien erreicht.

Der Einsatz des erfindungsgemäßen Adhäsivs ist nicht generell auf dem Dentalbereich beschränkt, sondern ist immer dann einzusetzen, wenn eine harte Substanz mit elastischem Material permanent oder temporär verbunden werden soll. Beispielsweise kann der Haftvermittler auch bei der Herstellung von Metall-Gummidichtungen in der Apparate- und Anlagentechnik eingesetzt werden.

Die Erfindung soll anhand nachstehender Ausführungsbeispiele näher erläutert werden, ohne sie einzuschränken.

### Beispiele

Adhäsiv 1: Aus Methyl-Methacrylat (MMA) und Hydroxymethyl-Methacrylat (HEMA) im Verhältnis 2:1 wird durch peroxidisch induzierte Polymerisation ein funktionalisiertes Copolymer erhalten. Hiervon werden 8 Gew.-Teile in 90 Gew.-Teilen 2-Butanon gelöst. Nach optionaler Zugabe eines Tensids werden 2 Gew.-Teile eines Silikonvernetzers mit mindestens 2 SiH-Gruppen (z.B. Hanse Vernetzer 200, Hanse Chemie, Geesthacht) zugesetzt. Nach 20 h Rühren ist die Umsetzung der Komponenten abgeschlossen.

Adhäsiv 2: 8 Gew.-Teile eines handelsüblichen Polyurethans (z.B. der Bayer AG, Leverkusen) werden in 90 Gew.-Teilen 2-Butanon gelöst. Nach optionaler Zugabe eines Tensids werden 2 Gew.-Teile des Silikonvemetzers (z.B. Hanse Vernetzer 200, Hanse Chemie, Geesthacht) aus Adhäsiv 1 zugesetzt. Nach 20 h Rühren ist die Umsetzung der Komponenten abgeschlossen.

Adhäsiv 3: 50 Gew.-Teile Adhäsiv 1 werden mit 50 Gew.-Teilen Adhäsiv 2 homogen vermischt.

Adhäsiv 4: 10 Gew.-Teile Adhäsiv 1 werden mit 90 Gew.-Teilen Adhäsiv 2 homogen vermischt.

### Haftversuche:

### 1) Starre Materialien

Es werden 1 cm breite Stäbe folgender Materialien verwendet:
kalthärtendes Prothesenreparatur-Material (Paladur®, Firma Kulzer) = PMMA
lichthärtendes Löffelmaterial (Individo® Lux, Firma VOCO GmbH) = IL
V4A-Stahl = S
Polyamid = PA
Urethandiacrylat (Microbase®, Firma Dentsply/DeTrey) = MB

### 2) Silikone

Es kommen zum Einsatz:
additionsvernetzendes Abformsilikon (Contrast®, Firma VOCO GmbH) = C additionsvernetzendes Unterfütterungssilikon (Ufi Gel®C, Firma VOCO GmbH) = U

Die Materialien werden nach Herstellerangaben verarbeitet.

### 3) Vorbereitung der Prüfkörper

Die Klebefläche (1 cm x 1 cm) wird durch eine Markierung im Abstand von 1 cm vom Rand aus festgelegt. Die Kunststoffe werden folgendermaßen vorbereitet: Oberflächenanrauhung durch Sandstrahlen mit Edelkorund, Säubern der Oberfläche mit Isopropanol. Die Stahlstäbe werden lediglich mit Isopropanol abgewischt. Anschließend wird das Adhäsiv in dünner Schicht aufgetragen. Nach 1 Minute kann Silikon auf die zu verklebende Fläche aufgebracht werden. Nun wird der mit Silikon beschichtete Prüfkörper in Linie so auf einen zweiten mit Adhäsiv versehenen Stab gepreßt, daß eine überlappende Verklebung mit einer Silikonschichtdicke von ca. 0,1 mm resultierte. Das Silikon wird anschließend nach Herstellerangaben ausgehärtet. Überschüssiges Material wird mit einem Skalpell entfernt.

### 4) Durchführung und Auswertung

Als Prüfgerät dient eine Universalprüfmaschine der Firma Zwick, versehen mit Zugspannklammern. Die Prüfkörper werden vorsichtig eingespannt und mit einer Geschwindigkeit von 5 mm/Min. auseinandergezogen. Aus jeweils fünf Prüflcörpern wird der Mittelwert für die Scherfestigkeit ermittelt.

### 5) Ergebnisse

| Beispiel-Nr. | Prüfkörper aus | Silikon | Adhäsiv | Scherfestigkeit |
|---|---|---|---|---|
| 1 | PMMA | U | 1 | 120 N/Cm² |
| 2 | PMMA | U | 2 | 82 N/cm² |
| 3 | PMMA | U | 3 | 81 N/cm² |
| 4 | PMMA | U | 4 | 157 N/cm² |
| 5 | IL | C | 1 | 24 N/cm² |
| 6 | IL | C | 2 | 10 N/cm² |
| 7 | IL | C | 3 | 8 N/cm² |
| 8 | IL | C | 4 | 7N/cm² |
| 9 | S | C | 1 | 25 N/cm² |
| 10 | S | C | 2 | 81 N/cm² |
| 11 | S | C | 3 | 58 N/cm² |
| 12 | S | C | 4 | 78 N/cm² |
| 13 | PA | C | 2 | 135 N/cm² |
| 14* | MB | U | 1 | 125 N/cm² |
| 15* | MB | U | 4 | 285 N/cm² |

| | | | | |
|---|---|---|---|---|
| *Zugversuch nach 24h Wasserlagerung bei 37 °C | | | | |

Zur besseren Ansicht und Veranschaulichung ist das Ergebnis dieser Haftuntersuchungen graphisch dargestellt (s. Grafik 1).

Anhand der in den Beispielen dargestellten Mischungsreihe kann beispielsweise die Lösung eines Haftproblems einfach abgelesen werden. Wird beispielsweise eine Haftvermittlung zwischen Metall (Stahl) und A-Silikon für eine zeitlich begrenzte Haftung mit Haftwerten zwischen 40 bis 60 N/cm² gesucht, so genügt ein Blick auf das Haftprofil (s. Grafik 1) um die Auswahl eines geeigneten Adhäsivs zu treffen (hier: Adhäsiv 3).

Die Beispiele zeigen sehr deutlich, daß die Haftkräfte des erfindungsgemäßen Haftvermittlers nach dem Baukastenprinzip für die verschiedensten Materialien individuell einstellbar sind und darüber hinaus einen kraftschlüssigen und zuverlässigen Hanverbund sicherstellen.

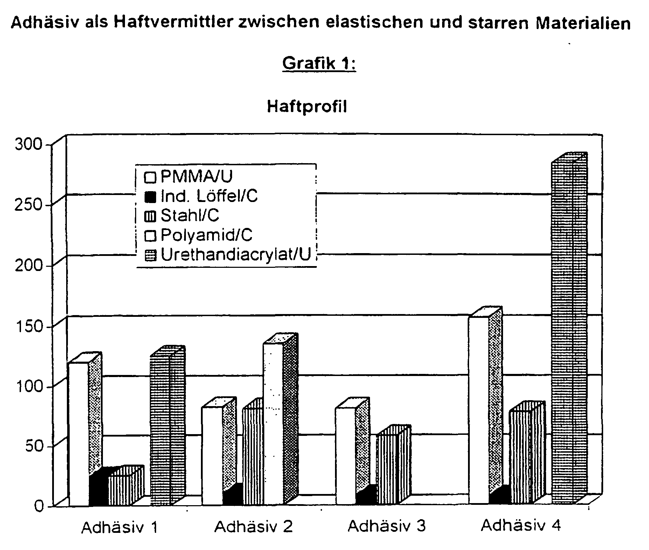

## Patentansprüche

1. Verfahren zum Herstellen eines Adhäsivs, **dadurch gekennzeichnet, daß** in einem Lösungsmittel eine oder mehrere Polymerverbindungen enthaltend ein MMA/HEMA Copolymer mit einem mindestens zwei SiH-Gruppen aufweisenden Silikonvernetzer umgesetzt werden.

2. Verfahren nach Anspruch 1, **dadurch gekennzeichnet, daß** ein erstes Adhäsiv aus einer Umsetzung eines ein MMA/HEMA Copolymer mit einem mindestens zwei SiH-Gruppen aufweisenden Silikonvemetzer und ein zweites Adhäsiv aus einer Umsetzung mit oder ohne Katalysator eines Polyurethans mit einem mindestens zwei SiH-Gruppen aufweisenden Silikonvemetzer gemischt werden.

3. Verfahren nach Anspruch 2, bei dem die Mischung aus etwa 50 bis 10 Gewichtsteilen des ersten Adhäsivs und im übrigen aus dem zweiten Adhäsiv besteht.

4. Verfahren nach einem der Ansprüche 1 bis 3, **dadurch gekennzeichnet, daß** das Lösungsmittel zu halogenierten Kohlenwasserstoffen, Alkoholen, Ketonen, Estern oder aromatische Verbindungen oder einer Mischung daraus gehört.

5. Verfahren nach einem der Ansprüche 1 bis 4, **dadurch gekennzeichnet, daß** das Lösungsmittel zusätzlich Additive, Stabilisatoren und/oder Füllstoffe enthält.

6. Verfahren zum Herstellen eines Adhäsivsystems, **dadurch gekennzeichnet, daß** verschiedene Haftvermittlungseigenschaften aufweisende Adhäsive gemäß den Ansprüchen 1 bis 5 hergestellt werden.

7. Verwendung eines gemäß einen der Ansprüche 1 bis 6 herstellbaren Adhäsivs, **dadurch gekennzeichnet, daß** es als Haftvermittler zwischen starren und elastischen Materialien eingesetzt wird, wobei das starre Material vorzugsweise Glas, Kunststoff oder Metall ist.

8. Verwendung nach Anspruch 7, **dadurch gekennzeichnet, daß** eine unbehandelte oder gegebenenfalls vorbehandelte Oberfläche aus starrem Material mit dem Adhäsiv bestrichen und nach Ablüften des Lösungsmittels das zum Verbund zu bringende elastische Material aufgetragen wird.

9. Verwendung nach Anspruch 8, **dadurch gekennzeichnet, daß** das elastische Material unmittelbar nach Ablüften des Lösungsmittels aufgetragen wird.

10. Verwendung nach einem der Ansprüche 7 bis 9, **dadurch gekennzeichnet, daß** die zu verbindenden Materialien ein Metall und ein A-Silikon sind.

11. Verwendung nach einem der Ansprüche 7 bis 10, **dadurch gekennzeichnet, daß** das Adhäsiv als Haftvermittler im Dentalbereich zur temporären Haftung von Abformmaterialien auf metallenen Abformlöffeln dient.

12. Verwendung nach einem der Ansprüche 7 bis 11, **dadurch gekennzeichnet, daß** die zu verbindenden Materialien ein PMMA-enthaltendes Material und ein bei Raumtemperatur aushärtendes (RTV) Silikon sind.

13. Verwendung eines Adhäsivs nach einem der Ansprüche 7, 8, 9 oder 12, **dadurch gekennzeichnet, daß** mittels des Adhäsivs im Dentalbereich Prothesen mit Silikonmaterialien permanent unterfüttert werden.

14. Verwendung nach einem der Ansprüche 7, 8, 9 oder 12, **dadurch gekennzeichnet, daß** das Adhäsiv als Haftvermittler im Dentalbereich zur temporären Haftung von Abformmaterialien auf individuellen Abformlöffeln dient.

## Claims

1. A method for producing an adhesive, **characterized in that** in a solvent, one or more polymeric compounds comprising an MMA/HEMA copolymer are reacted with a silicone cross linking agent comprising at least two SiH-groups.

2. The method according to claim 1, **characterized in that** a first adhesive from a conversion of an MMA/HEMA copolymer with a silicone cross linking agent comprising at least two SiH-groups is mixed with a second adhesive from a conversion, with or without a catalyst, of a polyurethane with a silicone cross linking agent comprising at least two SiH-groups.

3. The method according to claim 2, in which the mixture is composed of approximately 50 to 10 parts by weight of the first adhesive and the remainder of the second adhesive.

4. The method according to any of the claims 1 to 3, **characterized in that** the solvent belongs to halogenated hydrocarbons, alcohols, ketones, esters or aromatic compounds, or a mixture thereof.

5. The method according to any of the claims 1 to 4, **characterized in that** the solvent in addition contains additives, stabilizers and/or fillers.

6. A method for producing an adhesive system, **characterized in that** adhesives comprising different coupling agent characteristics are produced according to the claims 1 to 5.

7. Usage of an adhesive producible according to any of the claims 1 to 6, **characterized in that** it is used as coupling agent between rigid and elastic materials, wherein the rigid material is preferably glass, plastic, or metal.

8. Usage according to claim 7, **characterized in that** an untreated or if applicable a pretreated surface of a rigid material is coated with the adhesive and after evaporation of the solvent, the elastic material to be adhered is added.

9. Usage according to claim 8, **characterized in that** the elastic material is added immediately after evaporation of the solvent.

10. Usage according to any of the claims 7 to 9, **characterized in that** the materials to be connected are a metal and an A-silicone.

11. Usage according to any of the claims 7 to 10, **characterized in that** the adhesive serves as adhesive agent in the dental field for a temporary adhesion of moulding materials on metallic impression-taking trays.

12. Usage according to any of the claims 7 to 11, **characterized in that** the materials to be adhered are a material comprising a PMMA-containing material and a (RTV) silicone curing at room temperature.

13. Usage of an adhesive according to any of the claims 7, 8, 9 or 12, **characterized in that** by means of the adhesive, prostheses in the dental field are permanently padded with silicone materials.

14. Usage according to any of the claims 7, 8, 9 or 12, **characterized in that** the adhesive serves as coupling agent in the dental field for a temporary adhesion of moulding materials on individual impression-taking trays.

## Revendications

1. Procédé de préparation d'un adhésif, **caractérisé en ce qu'**on fait réagir, dans un solvant, un ou plusieurs composés polymères contenant un copolymère MMA/HEMA avec un agent réticulant de silicone comportant au moins deux groupes SiH.

2. Procédé selon la revendication 1, **caractérisé en ce qu'**on mélange un premier adhésif obtenu par réaction d'un copolymère MMA/HEMA avec un agent réticulant de silicone comportant au moins deux groupes SiH et un deuxième adhésif obtenu par réaction d'un polyuréthane avec un agent réticulant de silicone comportant au moins deux groupes SiH en présence ou non d'un catalyseur.

3. Procédé selon la revendication 2, dans lequel le mélange est constitué d'environ 50 à 10 parties en poids du premier adhésif, et dans une proportion restante du second adhésif.

4. Procédé selon une des revendications 1 à 3, **caractérisé en ce que** le solvant est choisi dans le groupe constitué des hydrocarbures halogénés, des alcools, des cétones, des esters et des composés aromatiques ou est un mélange d'entre eux.

5. Procédé selon une des revendications 1 à 4, **caractérisé en ce que** le solvant contient en plus des additifs, des agents stabilisants et/ou des charges.

6. Procédé de préparation d'un système adhésif, **caractérisé en ce qu'**on prépare selon les revendications 1 à 5 des adhésifs présentant des propriétés d'adhésion différentes.

7. Utilisation d'un adhésif pouvant être préparé selon une des revendications 1 à 6, **caractérisée en ce qu'**on l'utilise en tant qu'agent d'adhésion entre des matériaux rigides et des matériaux souples, le matériau rigide étant de préférence du verre, de la matière plastique ou du métal.

8. Utilisation selon la revendication 7, **caractérisée en ce qu'**on enduit avec l'adhésif une surface non-traitée ou éventuellement pré-traitée constituée d'un matériau rigide et, après évaporation du solvant, on applique le matériau souple à assembler.

9. Utilisation selon la revendication 8, **caractérisée en ce qu'**on applique le matériau souple immédiatement après évaporation du solvant.

10. Utilisation selon une des revendications 7 à 9, **caractérisée en ce que** les matériaux à joindre sont un métal et un silicone A.

11. Utilisation selon une des revendications 7 à 10, **caractérisée en ce que** l'adhésif sert, à titre d'agent d'adhésion, dans le domaine dentaire, à faire adhérer temporairement les matériaux de moulage sur des cuillers de moulage métalliques.

12. Utilisation selon une des revendications 7 à 11, **caractérisée en ce** les matériaux à assembler sont un matériau contenant du PMMA et un silicone durcissant à température ambiante (vulcanisant à température ambiante).

13. Utilisation selon une des revendications 7, 8, 9 ou 12, **caractérisée en ce qu'**on garnit à titre permanent, dans le domaine dentaire, des prothèses avec des matériaux de silicone au moyen de l'adhésif.

14. Utilisation selon une des revendications 7, 8, 9 ou 12, **caractérisée en ce que** l'adhésif sert, à titre d'agent d'adhésion, dans le domaine dentaire, à procurer une adhérence temporaire de matériaux de moulage sur des cuillers de moulage individuelles.
